Europäisches Patentamt

European Patent Office

Office européen des brevets

(11) Numéro de publication: **0 425 361 A1**

(12) **DEMANDE DE BREVET EUROPEEN**

(21) Numéro de dépôt: 90402969.1

(22) Date de dépôt: 22.10.90

(51) Int. Cl.⁵: **A61M 16/04, A61M 25/04**

(30) Priorité: 23.10.89 FR 8913858

(43) Date de publication de la demande:
**02.05.91 Bulletin 91/18**

(84) Etats contractants désignés:
**DE ES GB IT**

(71) Demandeur: **OVI**
**Site Montesquieu, Bordeaux Technopolis**
**F-33650 Martillac(FR)**

(72) Inventeur: **Boudey,Christian**
**Hôpital Pellegrin Place Amélie Raba Léon**
**F-33076 Bordeaux Cédex(FR)**

Inventeur: **Cros Anne-Marie**
**Hôpital Pellegrin Place Amélie Raba Léon**
**F-33076 Bordeaux Cédex(FR)**
Inventeur: **Guenard Hervé Laboratoire de**
**Physiologie**
**Université de Bordeaux II 146 rue Léo**
**Saignat**
**F-33076 Bordeaux Cédex(FR)**

(74) Mandataire: **Grosset-Fournier, Chantal**
**Catherine et al**
**ERNEST GUTMANN-YVES PLASSERAUD S.A.,**
**67 boulevard Haussmann**
**F-75008 Paris(FR)**

(54) **Catheter pour la ventilation artificielle intra-pulmonaire d'un patient.**

㊼ Catheter pour la ventilation artificielle intra-pulmonaire d'un patient, comportant un tube allongé et de faible diamètre (2), en matériau plastique, propre à être réuni à l'extérieur de la trachée (10) du patient à un dispositif de ventilation pour l'injection d'air ou d'un mélange respiratoire gazeux approprié, et dont l'extrémité distale (4) est amenée en un point donné écarté de la carena 11.

Selon l'invention, ce catheter se caractérise en ce qu'il comporte un organe de centrage (5) vis-à-vis de la paroi interne (14) de la trachée, porté par le catheter et adapté à s'expanser radialement par rapport à la surface externe du catheter afin de venir ponctuellement en contact avec la paroi en regard de la trachée selon une pluralité de zones d'appui régulièrement réparties autour de la trachée.

FIG. 2

Xerox Copy Centre

La présente invention est relative à un catheter de faible dimension transversale, destiné notamment à permettre une ventilation artificielle d'un patient, en particulier lors d'une opération chirurgicale pratiquée sur le pharynx, le larynx ou la trachée de celui-ci.

On sait que pour faciliter l'intervention du chirurgien sur une partie quelconque de la trachée ou du larynx d'un malade, il est généralement nécessaire de réaliser une ventilation pulmonaire articielle de ce dernier, un injectant directement dans ses poumons un flux d'air ou un mélange respiratoire approprié sous une pression et avec une fréquence déterminées. En particulier, un tube ou catheter de petit diamètre, de l'ordre de 1 à 2,5 mm, introduit par la bouche ou les voies nasales d'un patient permet d'injecter, sous une pression comprise entre 1 et 4 bars, un débit satisfaisant, en évitant notamment l'usage d'une sonde de plus gros diamètre, difficile à mettre en place et que la patient supporte moins bien, alors qu'un tube de petit diamètre permet au contraire de laisser libre un espace suffisant entre le tube et la trachée pour le passage des instruments nécessaires à l'opération envisagée, par exemple une sonde laser ou tout autre dispositif indispensable à la bonne exécution de l'intervention en cours. La fréquence d'insufflation de l'air ou du mélange respiratoire, de l'ordre généralement de 1 à 5 Hz, permet de diminuer d'autant la pression trachéale, celle-ci restant cependant suffisamment grande pour vaincre la résistance des voies aériennes.

En outre, par l'effet d'entraînement créé par l'énergie du gaz à la sortie du tube, le débit fourni assure une ventilation appropriée des poumons dans des conditions optimales, le volume reçu par les poumons étant égal à la somme du volume injecté par le catheter et du volume entraîné à l'extérieur de celui-ci dans la trachée, ce volume pouvant représenter environ les 2/3 du total.

D'autres paramètres sont également déterminants pour réaliser une ventilation forcée satisfaisante, qui dépend selon le cas, du diamètre relatif du catheter vis-à-vis de celui de la trachée, de l'efficacité des valves du distributeur de ventilation fournissant le mélange respiratoire sous pression de la longueur du catheter, surtout de la position de l'extrémité de celui-ci pénétrant dans la trachée par rapport à l'emplacement des cordes vocales d'une part et de la carena d'autre part, c'est-à-dire de l'embranchement des bronches sur la trachée constituant la bifurcation trachéale reliant celle-ci aux poumons, afin notamment d'éviter que le jet gazeux ne frappe la paroi de la trachée, en créant un reflux inspiratoire gênant l'effet d'entraînement, et enfin des caractéristiques pulmonaires (débit, volume, rythme respiratoire, etc...) du patient lui-même.

Enfin, il s'avère qu'une condition essentielle à la réalisation d'une ventilation intra-pulmonaire adéquate réside dans la faculté de centrer aussi parfaitement que possible le catheter, sensiblement dans l'axe de la trachée.

Or, les catheters actuellement en usage en laryngoscopie, avec ou sans microchirurgie, du genre tube de Carden ou catheter transcricoïdien de Ravussin, ou plus simplement encore du type sonde d'aspiration, généralement en chlorure de polyvinyle, le cas échéant avec protection par une bande d'aluminium enroulée, présentent des inconvénients pratiques, soit parce qu'ils ne permettent pas un entraînement satisfaisant du mélange respiratoire, voire provoquent un reflux préjudiciable ou même une rétention d'air qui freinent l'expiration du patient, avec des risques de distension pulmonaire. Dans le cas d'un catheter transcricoïdien, le dispositif exige un percement de la trachée avec un trocart pour le passage du catheter, d'où il résulte au moins une plaie et un traumatisme, qui rendent son emploi peu adapté à toutes les circonstances.

La présente invention concerne un catheter pour ventilation intra-pulmonaire qui évite ces inconvénients, qui soit simple à fabriquer, facile à mettre en place, soit peu coûteux et surtout permette d'assurer son centrage efficace dans la trachée en toutes circonstances, tout en laissant libre entre la paroi de celle-ci et le catheter, un jeu approprié pour le passage des instruments opératoires nécessaires, en particulier une sonde laser, notamment à $CO_2$, sans présenter de risques d'inflammation sous l'effet du rayonnement fourni.

A cet effet, le catheter considéré, comportant un tube allongé et de faible diamètre, en matériau plastique, propre à être réuni à l'extérieur de la trachée du patient à un dispositif de ventilation pour l'injection d'air ou d'un mélange respiratoire gazeux approprié, et dont l'extrémité distale est amenée en un point donné écarté de la carena, se caractérise en ce qu'il comporte un organe de centrage vis-à-vis de la paroi interne de la trachée, porté par le catheter et adapté à s'expanser radialement par rapport à la surface externe du catheter afin de venir ponctuellement en contact avec la paroi en regard de la trachée selon une pluralité de zones d'appui régulièrement réparties autour du catheter.

Avantageusement, l'organe de centrage est constitué par un petit élément de tube mince et souple, monté coaxialement autour du catheter, en contact avec la surface externe de celui-ci, l'une des extrémités de cet élément de tube étant solidarisée du catheter et l'autre pouvant coulisser sur celui-ci, de manière à ce que l'élément de tube se déforme symétriquement vers l'extérieur lorsque les deux extrémités sont rapprochées l'une de l'autre.

Selon une autre caractéristique, le petit élément de tube mince est fendu longitudinalement entre deux bordures prévues aux extrémités pour former des bandes voisines autour du catheter, de telle sorte que le rapprochement des extrémités de celui-ci provoque la flexion des bandes jusqu'à appui de leur partie médiane sur la paroi interne de la trachée.

Le catheter, muni de son élément de tube mince et souple, peut ainsi être facilement introduit dans la trachée du patient jusqu'à ce que son extrémité distale soit exactement localisée dans celle-ci, après que la bordure postérieure de l'élément, qui peut coulisser sur le catheter ne soit repoussée vers la bordure antérieure solidarisée du catheter, au moyen d'une pince ou autre élément analogue, en faisant alors saillir les bandes voisines ménagées dans l'élément de tube qui assurent le cantrage du catheter lorsqu'elles viennent en contact avec la paroi en regard de la trachée.

Avantageusement et selon une autre caractéristique, le nombre de bandes voisines, réalisées dans l'élément de tube mince et souple fendu, est égal à quatre au moins et éventuellement est supérieur.

De préférence et selon une autre caractéristique intéressante de l'invention, le catheter et l'élément de tube fendu sont réalisés en "teflon" (polytetrafluoroéthylène) ou en tout autre matériau plastique équivalent, présentant une rigidité suffisante de façon à éviter le battement sous l'effet du flux dans la catheter. A noter que l'usage de teflon présente également l'avantage d'être ininflammable sous l'effet d'un faisceau laser, notamment issu d'une sonde à CO2.

D'autres caractéristiques d'un catheter pour la ventilation intra-pulmonaire d'un patient, établi conformément à l'invention apparaîtront encore à travers la description qui suit d'un exemple de réalisation, donné à titre indicatif et non limitatif, en référence au dessin annexé sur lequel :

- La Figure 1 est une vue schématique du catheter de ventilation considéré, muni de son organe de centrage, préalablement à son déploiement.

- La Figure 2 représente le même catheter une fois mis en place, avec son organe de centrage déployé dans la trachée pour permettre le maintien du catheter sensiblement dans l'axe de celle-ci.

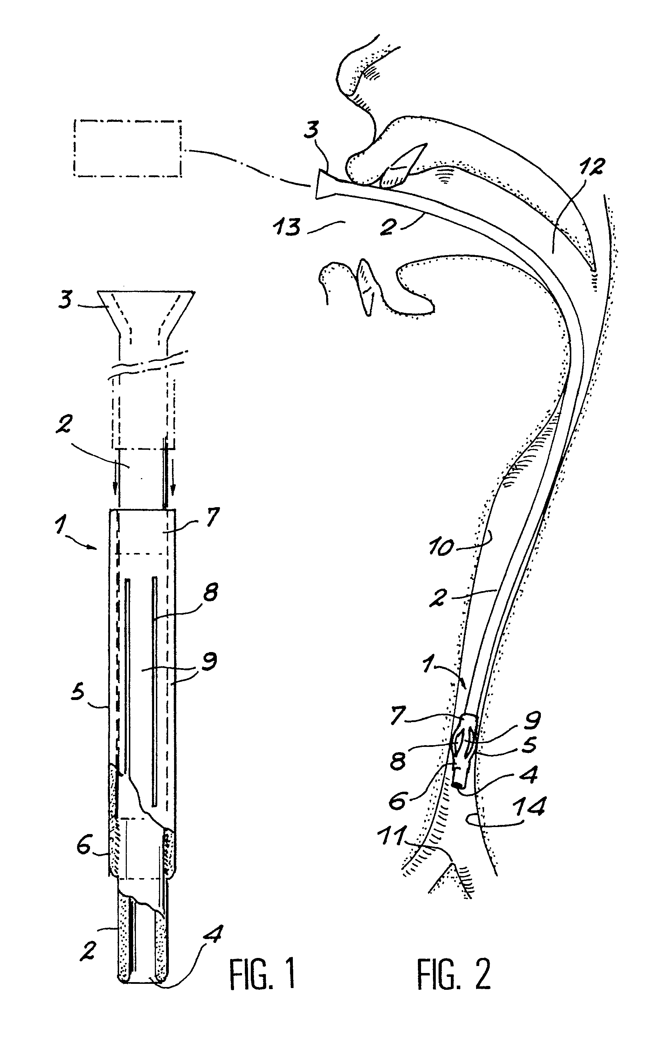

Sur la Figure 1, le catheter de ventilation représenté, désigné dans son ensemble sous la référence 1, est constitué par un tube fin 2, de préférence réalisé en un matériau plastique présentant une souplesse relative acceptable, notamment du genre polytetrafluoroéthylène (Teflon). Le tube 2 présente une longueur suffisante pour pourvoir être ultérieurement introduit dans la trachée du patient, cette longueur étant naturellement variable selon que ce dernier est un adulte ou un enfant. De même, le diamètre interne du tube peut être adapté selon les utilisations envisagées, en restant compris en règle générale, entre 1 et 2,5 mm au plus.

A son extrémité supérieure, destinée notamment à émerger de la bouche du patient, le tube 2 comporte un raccord 3 permettant de le relier à une installation de ventilation, très schématiquement représentée sur la Figure 2 et susceptible de fournir un mélange gazeux respiratoire approprié, sous une pression qui également dépend des conditions, d'utilisation et qui notamment peut être comprise entre 1 et 4 bars. L'installation de ventilation permet par ailleurs de faire varier la fréquence d'injection du mélange respiratoire injecté dans le tube 2, cette fréquence étant comprise entre 1 et 5 Hz généralement.

Conformément à l'invention, le tube 2 est muni, à écartement convenable de son extrémité distale 4 qui pénètre dans la trachée du patient pour être amenée au voisinage de la carena, c'est-à-dire de l'embranchement des bronches avec la trachée, d'un organe de centrage constitué d'un petit élément de tube souple 5, de préférence réalisé en matière plastique et dans le même matériau que celui qui constitue le tube 2.

Cet élément de tube 5 comporte à ses extrémités deux bordures, respectivement 6 et 7, formant colliers autour du tube 2, la bordure 6 située au plus près de l'extrémité distale 4 étant solidarisée de la paroi externe du tube 2, par thermosoudure, collage ou autre moyen équivalent.

L'autre bordure 7 est en revanche disposée sur le tube 2 en entourant celui-ci mais en pouvant coulisser légèrement contre sa surface externe, de façon à pouvoir se rapprocher légèrement de la bordure 6. L'élément de tube 5 comporte par ailleurs, entre ses bordures 6 et 7, un ensemble de fentes longitudinales 8, régulièrement réparties autour de l'axe du tube 2 et délimitant deux à deux des bandes étroites 9, parallèles et successives.

De préférence, l'élément de tube 5 présente une dimension longitudinale de l'ordre de 3 à 4 cm, le nombre des fentes 8 et par suite des bandes 9 qu'elles délimitent, étant égal au moins à quatre, mais le cas échéant à six ou huit.

La Figure 2 illustre, une fois le catheter 1 équipé de son élément de tube 5 tel que décrit ci-dessus, mis en place dans la trachée 10 du patient, la façon dont est utilisé cet organe de centrage pour maintenir le catheter sensiblement dans l'axe de la trachée, avec son extrémité distale 4 convenablement écartée de la carena 11.

En agissant sur la bordure coulissante 7 de l'élément 5, en la repoussant légèrement vers le bas en direction de la bordure fixe 6, selon le sens de la flèche représentée. Les bandes étroites 9 de l'élément de tube 5 font progressivement saillie vers l'extérieur jusqu'à paraître adaptables au diamètre de la trachée du malade à ventiler, en maintenant alors le catheter 1 parfaitement centré dans celle-ci.

La ventilation du patient peut alors être réalisée dans des conditions optimales, en permettant notamment un bon entraînement de l'air ou du mélange respiratoire autour du catheter et un reflux expiratoire du patient sans gêne particulière, les bandes 9 très étroites n'opposant au débit ainsi expiré qu'une résistance très faible.

Bien entendu et comme il résulte déjà de ce qui précède, il va de soi que l'invention ne se limite pas à l'exemple de réalisation plus spécialement décrit et représenté ci-dessus ; elle en embrasse au contraire toutes les variantes. En particulier, il est clair que le tube 2 constituant le catheter proprement dit aussi bien que l'élément de tube 5 monté coaxialement sur ce dernier pour assurer son centrage de la façon indiquée, pourraient être réalisés en un autre matériau dont les caractéristiques de souplesse relative permettent son introduction facile dans la trachée jusqu'au niveau souhaité, un tel tube étant en outre ininflammable sous l'effet d'une sonde laser notamment.

## Revendications

1 - Catheter pour la ventilation artificielle intrapulmonaire d'un patient, comportant un tube allongé et de faible diamètre (2), en matériau plastique, propre à être réuni à l'extérieur de la trachée (10) du patient à un dispositif de ventilation pour l'injection d'air ou d'un mélange respiratoire gazeux approprié, et dont l'extrémité distale (4) est amenée en un point donné écarté de la carena 11, caractérisé en ce qu'il comporte un organe de centrage (5) vis-à-vis de la paroi interne (14) de la trachée, porté par le catheter et adapté à s'expanser radialement par rapport à la surface externe du catheter afin de venir ponctuellement en contact avec la paroi en regard de la trachée selon une pluralité de zones d'appui régulièrement réparties autour de la trachée.

2 - Catheter selon la revendication 1, caractérisé en ce que l'organe de centrage (5) est constitué par un petit élément de tube mince et souple, monté coaxialement autour du catheter, en contact avec la surface externe de celui-ci, l'une des extrémités (6) de cet élément de tube étant solidarisée du catheter et l'autre (7) pouvant coulisser sur celui-ci de manière à ce que l'élément de tube se déforme symétriquement vers l'extérieur lorsque les deux extrémités sont rapprochées l'une de l'autre.

3 - Catheter selon la revendication 2, caractérisé en ce que le petit élément de tube mince (5) est fendu longitudinalement entre deux bordures (6, 7) prévues aux extrémités pour former des bandes (9) voisines autour du catheter, de telle sorte que le rapprochement des extrémités de l'élément provoque la flexion des bandes jusqu'à appui de leur partie médiane sur la paroi interne (14) de la trachée (10).

4 - Catheter selon la revendication 3, caractérisé en ce que le nombre de bandes voisines (9), réalisées dans l'élément de tube mince (5) fendu et souple est égal à quatre au moins et est éventuellement supérieur.

5 - Catheter selon l'une quelconque des revendica1 à 4, caractérisé en ce que le tube (2) et l'organe de centrage (5) sont réalisés en polytetrafluoroéthylène.

FIG. 1    FIG. 2

Office européen
des brevets

RAPPORT DE RECHERCHE
EUROPEENNE

Numéro de la demande

EP 90 40 2969

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | DE-A-2 709 302   (ZEPPELIN)<br>* Page 5, ligne 9 - page 6, ligne 5; figure 1 *<br>– – – | 1-4 | A 61 M 16/04<br>A 61 M 25/04 |
| A | US-A-4 608 965   (ANSPACH)<br>* Colonne 1, ligne 65 - colonne 2, ligne 31; figures 1-3 *<br>– – – | 1-4 | |
| A | US-A-3 585 983   (KANTROWITZ)<br>* Colonne 3, lignes 13-28 *<br>– – – | 5 | |
| A | EP-A-0 204 500   (PFIZER HOSPITAL)<br>* Page 6, lignes 3-19; page 8, lignes 1-13 *<br>– – – – – | 5 | |

DOMAINES TECHNIQUES
RECHERCHES (Int. Cl.5)

A 61 M
A 61 B

Le présent rapport de recherche a été établi pour toutes les revendications

| Lieu de la recherche | Date d'achèvement de la recherche | Examinateur |
|---|---|---|
| La Haye | 17 décembre 90 | SCHOENLEBEN J.E.F. |

CATEGORIE DES DOCUMENTS CITES

X : particulièrement pertinent à lui seul
Y : particulièrement pertinent en combinaison avec un
  autre document de la même catégorie
A : arrière-plan technologique
O : divulgation non-écrite
P : document intercalaire
T : théorie ou principe à la base de l'invention

E : document de brevet antérieur, mais publié à la
  date de dépôt ou après cette date
D : cité dans la demande
L : cité pour d'autres raisons

&: membre de la même famille, document
  correspondant